# EUROPEAN PATENT SPECIFICATION

(11) **EP 0 536 366 B1**
(45) Date of publication and mention of the grant of the patent: **19.06.1996**
(21) Application number: 92908997.7
(22) Date of filing: 01.04.1992
(51) Int. Cl.: A61M 5/315

(54) **INJECTION DEVICE**
INJEKTIONSVORRICHTUNG
DISPOSITIF POUR INJECTIONS

(30) Priority: 25.04.1991 SE 9101262
(43) Date of publication of application: 14.04.1993
(73) Proprietor: Pharmacia AB (reg.number 556131-9608), S-171 97 Stockholm (SE)
(72) Inventor: HJERTMAN, Birger, S-162 43 Vällingby (SE); LEVANDER, Gustav, S-161 28 Bromma (SE); LJUNGQUIST, Olle, S-183 67 Täby (SE)
(74) Representative: Onn, Thorsten
(86) International application number: SE9200206
(87) International publication number: WO9219297

(56) References cited:
- EP-A- 0 293 572
- EP-A- 0 327 910
- WO-A-90/07946

## Description

The present invention relates to the field of devices for parenteral injections, and more specifically to a device for dosing and administering injections from an injection cartridge.

Injection cartridges have found a wide use in the field of parenteral injections. Such cartridges usually consist of a tubular container of glass or plastic material for the preparation to be injected. At its front end, the cartridge is usually shaped like a bottleneck, and is closed by a septum of rubber or a suitable plastic material. This septum is to be pierced by an injection needle when an injection is to be administered from the cartridge. Furthermore, the septum is secured in place by a metal capsule, which is crimped around a flange at the neck of the cartridge and which is provided with an opening in its centre portion, so that the septum is exposed and may be pierced by the needle. At its rear end, the cartridge is closed by a stopper of rubber or a plastic material. This stopper acts as a piston to be pushed forward for expelling the preparation from the cartridge through the needle.

In use, the cartridge is placed in a suitably adapted holder, which is provided at its front end with an opening which gives access to the front end of the cartridge and to which can be connected a needle for piercing the septum of the cartridge. The holder is also provided with a plunger device, which acts on the piston at the rear end of the cartridge, to urge the piston forwards for expelling the preparation from the cartridge.

Injection cartridges of the dual-chamber type are also known. These cartridges are intended for preparations which are not sufficiently stable to be stored for a long time in their ready-to-use state. Such cartridges are divided into two chambers, each of which contains a component of the preparation, usually a solid component and a solvent therefor. Through a suitable arrangement of pistons and bypass channels, the two components may be mixed immediately before use.

Injection cartridges of the types described above have found a wide acceptance because of their important advantages. They are very easy to handle in use, and are more safe from contamination than prior art syringes, where the user has to fill the syringe by drawing up liquid from a storage bottle. Because of this ease and safety in handling, injection cartridges have found a wide use in applications where the patient has to administer injections to himself, such as in the treatment of diabetics with insulin, or in prolonged treatments with growth hormones. Various types of devices for such injections are known, for example from the publications EP-A1-293 572, EP-A2-327 910, WO 90/07946, and others.

The preparation in an injection cartridge is usually not intended to be administered in a single dose, but rather in a number of separate doses. Also, different patients often require different doses. Because of this, there exists a need for accurate metering and administering of a plurality of doses of a preparation from one single injection cartridge.

A number of devices for dosing and administering a preparation from an injection cartridge are previously known, for instance from the above-mentioned publications. However, there is still room for improvement in this field. A number of the prior art devices are of a complicated construction, which makes them expensive and often cumbersome for the patient to use. In some of the known devices, it is also difficult to determine correctly the dose which has been set. Furthermore, many of the known devices are deficient from a safety point of view. Thus, there is usually no provision to prevent that an excessively high dose is set and administered, which may have very serious consequences. Also, for safety reasons, it is often desirable that it should not be possible to re-use the device with a new cartridge.

The above disadvantages are eliminated by the injection device of the present invention. The device consists of a small number of parts, which are easily assembled and operated, and when a dose has been set, it can be read simply and unambiguously. Furthermore, it is not possible to set the device at an excessively high dose, and when a cartridge has been emptied, it will not be possible to disassemble the device to make it operable with a new cartridge.

According to the invention, there is provided a device for dosing and administering one or more injections from an injection cartridge, said device comprising a front tubular sleeve which encloses the cartridge and at its front end is arranged to receive an injection needle to establish a connection with the interior of the cartridge, and at its rear end is provided with an interior thread; a rear tubular sleeve, which at its front end has an exterior thread which cooperates with the interior thread of the front sleeve, and at its rear end is closed by a transversal end wall having a through-going hole; and a piston rod, which is arranged essentially within the rear sleeve, with its front end secured to the piston of the injection cartridge, and its rear end protruding through the hole in the rear end wall of the rear sleeve, said piston being arranged to be moved forwards to urge the piston of the injection cartridge forward, thereby to expel the preparation from the cartridge through the needle. In one of the sleeves are arranged a number of axial grooves, with a regular angular spacing around the periphery of said sleeve, and in the other sleeve is arranged at least one radial projection, which cooperates resiliently with said axial grooves in the first sleeve to act as a pawl-and-ratchet mechanism which only allows rotation in one direction when the front and the rear sleeves are screwed together, and which indicates audibly when the sleeves have been rotated by a given angle in regard to each other. What characterizes the invention is that said grooves and said at least one projection are arranged in the threaded parts of the sleeves, and that the piston rod at its rear end is provided with indicating means which indicate any displacement between the rear sleeve and the piston rod in relation to each other in the axial direction, thereby also indicating the dose set to be administered. The rear sleeve is provided with a tubular extension which extends behind its rear end wall and is provided with an opening or window in its side wall, through which the indicating means at the rear end of the piston may be observed.

A device according to the preamble of claim 1 is previously known from the above-mentioned WO-A-90/07946.

In a preferred embodiment, the piston rod is provided with stopping means, which cooperate with means arranged within the rear sleeve to prevent the piston rod and the rear sleeve from being displaced in relation to each other by more than a predetermined length.

The invention will be described in more detail in the following specification, in connection with the appended drawings.

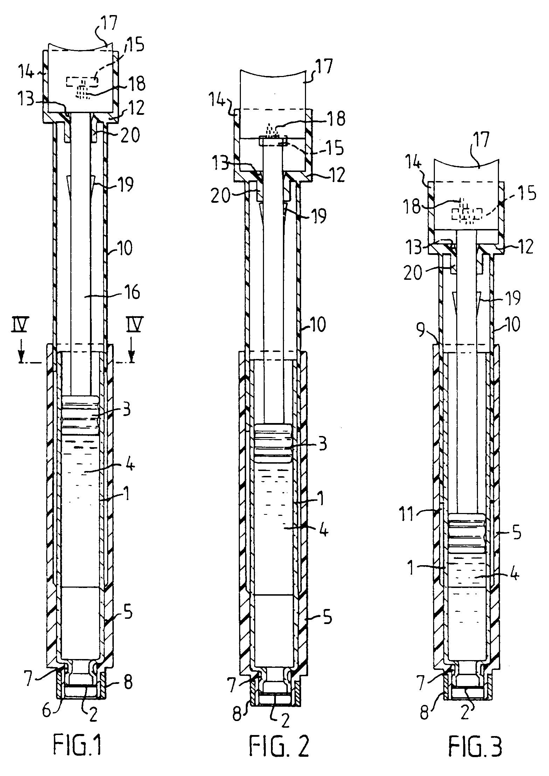

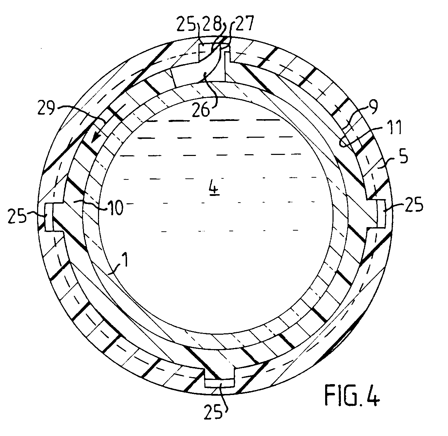

In the drawings, Figure 1 shows a shematical sectional view of an injection device of the invention before a dose has been set and administered. Figure 2 shows the same device after a dose has been set but before the administering thereof. Figure 3 shows the device after a number of doses have been administered. Figure 4 shows a sectional transversal view along IV-IV in Figure 1.

In Figure 1, the device is shown containing an injection cartridge 1, which is shaped like a bottleneck at its front end 2, and is closed at this end by a rubber septum held in place by a metal capsule. At its rear end, the cartridge is closed by a piston 3, which may be moved forwards to expel the preparation 4 which is filled into the cartridge. The injection cartridge is conventional in its design, and does therefore not have to be described in closer detail.

The cartridge is enclosed in a front tubular sleeve 5. At its front end, this sleeve has a neck 6 with an opening and an internal flange 7, against which rests the shoulder portion of the front end 2 of the cartridge 1. The neck 6 is provided with a thread 8 arranged to accept an injection needle or cannula of a conventional type (not shown). When the needle is attached, it will penetrate the septum of the cartridge 1 to establish a liquid connection with its interior.

The front sleeve 5 has such a length that it extends slightly beyond the rear end of the cartridge 1. At its rear end, the front sleeve is provided with an internal thread 9, which has axial grooves, as will be described in more detail below.

A rear tubular sleeve 10 having an external thread 11 is screwed into the front sleeve 5 by means of said internal thread 9. At its rear end, the rear sleeve is closed by a transversal end wall 12, which has a through-going hole 13. The length of the rear sleeve 10 between its front end and the transversal wall 12 should be at least equal to the maximum stroke of the piston 3 in the injection cartridge 1. The rear sleeve is arranged to be screwed into the front sleeve 5 between said front sleeve and the cartridge 1, and the diameters of the front and rear sleeves should be dimensioned accordingly. Also, the internal thread 9 of the front sleeve 5 has such a length that it permits the rear sleeve 10 to be screwed into the front sleeve 5 a distance corresponding to the maximum stroke of the piston 3 of the cartridge 1. At the front end of the front sleeve 5, its internal diameter is reduced such that the front end of the cartridge 1 fits snugly in said sleeve. The cartridge 1 is intended to be inserted into the front sleeve 5 through the opening at its rear end, which means that the front end part of the front sleeve 5 can be integral with said sleeve.

Beyond the transversal end wall 12 of the rear sleeve 10 is arranged a tubular extension 14. This extension is provided with an opening 15, which serves as a window, through which the dose set may be read, as will be explained below.

Within the rear sleeve 10 is arranged a piston rod 16, which serves to act on the piston 3 of the cartridge 1 for expelling the injectable preparation 4 from the cartridge. This piston rod 16 is secured to the rear face of the piston 3 by means of a snap mechanism, thread, adhesive or the like. The piston rod 16 extends rearwards within the rear sleeve 10 through the hole 13 in the transversal wall 12, and is terminated by a thicker cylindrical part 17, which fits snugly but slidably in the tubular extension 14 of the rear sleeve 10. On the face of the thicker cylindrical part 17 are arranged indicia 18, which are aligned with the window 15 in the cylindrical extension 14, such that a selected part thereof may be observed through said window. This serves as the indicating means to show the dose set to be administered.

The piston rod 16 is also provided with lugs 19 at a predetermined distance from the thicker cylindrical part 17. These lugs are arranged to cooperate with a tubular extension 20 which extends forward from the front face of the transversal wall 12, such that they prevent a relative movement between the rear sleeve 10 and the piston rod 16 by more than a predetermined distance. In this way, it will not be possible to set an excessively high dose of the preparation to be administered.

Figure 4 shows a transversal sectional view along the line IV-IV in Figure 1. The injection cartridge 1 with the injectable preparation 4 is shown enclosed by the rear sleeve 10 and the front sleeve 5. As mentioned in the foregoing, the internal thread 9 of the front sleeve 5 is provided with axial grooves 25, of which four are shown in the figure. Other numbers of grooves are also possible. The grooves 25 are arranged around the internal periphery of the front sleeve with equal angular spacing and extend in the axial direction for a length which is at least equal to the maximum stroke of the piston 3 of the cartridge 1.

In the external thread 11 of the rear sleeve 10 near its front end is arranged a ratchet device, which is schematically shown at 26. This ratchet device consists of a resilient projection 26, which has an edge 27 and an inclined part 28. The projection 26 cooperates with the axial grooves 25 in such a way that the rear sleeve 10 may be screwed into the front sleeve 5 by rotating it into the direction shown by the arrow 29. During this rotation, the projection 26 will be bent inwards, but will by its resilience snap out into the axial groove 25 every time it passes such a groove. Because of said resilience, it will then be possible to continue the rotation in the direction of the arrow 29. However, a rotation in the opposite direction will not be possible, as the projection 26 will snap out into an axial groove 25, and its edge 27 will catch in said groove, making further rotation in that direction impossible. Thus, the arrangement shown will act as a pawl-and-ratchet mechanism, permitting relative rotation in one direction only. At the same time, the ratchet mechanism will make an audible click each time that a projection 26 snaps out into a groove 25 during a rotation in the permitted direction. By counting the number of clicks, the user may estimate the magnitude of the dose set.

The function of the device of the invention will now be described in detail, with reference to the drawings:
When the device is to be prepared for dosing and injection, an injection cartridge 1 is first inserted through the rear opening of the front sleeve 5, such that the shoulder of its neck portion 2 rests against the internal flange 7 of the front sleeve 5. The septum at the front end of the cartridge is exposed through the opening in the front end of the front sleeve 5, so that an injection needle may be connected to the cartridge. Near its front end, the front sleeve 5 has such an internal diameter that the cartridge 1 fits snygly within said sleeve.

The rear sleeve 10 and the piston rod 16 are then mounted in the front sleeve 5. The rear sleeve and the piston rod have previously been assembled together such that the rear part of the piston rod 16 goes through the hole 13 in the transversal wall 12 and the tubular extension 20, and because of the lugs 19, the piston rod 16 cannot be pulled out through the hole 13. The front end of the piston rod 16 is then attached to the rear surface of the piston 3 by means of a snap mechanism, an adhesive or the like, such means for attachment having been prepared in advance in the manufacture of the piston 3 and the piston rod 16.

The rear sleeve 10 is then screwed into the front sleeve 5 by means of the interior thread 9 and the exterior thread 11. At this screwing together, the projection 26 with its edge 27 will catch in the axial grooves 25 (Figure 4), such that it is possible to screw the rear sleeve into the front sleeve, but a relative rotation in the opposite direction becomes impossible due to the pawl-and-ratchet effect of the projection 26 and the axial grooves 25.

In this rotatory movement, the piston rod 16 will remain stationary and will not rotate. When the two sleeves 5 and 10 are screwed together, the piston rod 16 will be displaced in an axial direction in relation to the rear sleeve 10, such that the thickened cylindrical part 17 protrudes somewhat from the tubular extension 14.

Up to this point, the injection device may be assembled by the manufacturer or the user.

When the first dose is to be metered and administered from a fresh cartridge, the user first attaches an injection needle to the front end of the front sleeve 5 by means of the thread 8, such that a fluid connection is established with the interior of the cartridge 1 through the opening at the front end of the front sleeve 5. Usually holding the device with the needle pointing upwards, the user then pushes the thickened cylindrical part 17 of the piston rod 16 forwards, until it rests against the rear face of the transversal end wall 12. This will serve to expel possible air from the cartridge and to zero the dosage setting. The indicia 18 on the thickened cylindrical portion 17 are arranged such that a zero will be read through the opening or window 15 when the thickened cylindrical part 17 rests against the rear face of the transversal end wall 12. Thus, the dosing device may always be zeroed accurately.

To set a prescibed dose, the user then turns the rear sleeve 10 in the direction shown by the arrow 29 (Figure 4), such that the rear sleeve 10 is screwed into the front sleeve 5. As the piston rod 16 is attached to the piston 3 of the cartridge 1, it will not follow the rear sleeve 10 in its forward movement, but will instead protrude with its rear thickened portion 17 from the tubular extension 14 of the rear sleeve 10. The indicia 18 on this thickened portion are arranged such that when there has occured a relative displacement between the thickened portion 17 and the tubular extension 14 which corresponds to a certain dose, this dose can be read through the window 15.

Thus, the user screws the rear sleeve 10 into the front sleeve 5 until the indicia corresponding to the prescribed dose can be read through the window 15. At the same time, the pawl-and-ratchet mechanism 25, 26, 27, 28 is in function, so that the user will hear an audible click each time the projection 26 snaps out into an axial groove 25. By counting the number of clicks, the user can further estimate the magnitude of the dose set. Also, the pawl-and-ratchet mechanism prevents a relative rotation between the front sleeve 5 and the rear sleeve 10 in the direction opposite that shown by the arrow 29. If such a movement were possible, it would mean that the piston rod 16 with the piston 3 secured thereto could be pulled backwards, so that air would be sucked into the cartridge 1. This, of course, must be avoided.

When the prescribed dose has been set and an injection needle or cannula has been connected to the front end of the front sleeve 5 by means of the thread 8, the device is ready for administration by injection. To administer the dose set, the user pushes the thickened cylindrical portion 17 forward into the tubular extension 14 until it rests against the rear surface of the transversal end wall 12. By means of the piston rod 16, this forward movement will be translated to the piston 3, which will move forward a set distance in the cartridge 1 to expel a set amount of the injectable preparation 4 through the injection needle connected to the cartridge through the septum at its front end 2. When the prescribed dose has been administered, the reading through the window 15 will again be zero.

After administration, the injection needle is usually removed and discarded, and the device is then ready for the next setting and administering of a dose, which may be the same or different from the dose previously administered. This renewed setting and administration is carried out in the same way as previously described. A new needle is usually used for each injection. It can be attached to the front end of the front sleeve 5 before or after the dose has been set, as there should be no need to expel air from the cartridge and zero the dosing device after the first injection has been administered from the cartridge. Thus, one of the importand advantages of the device of the invention is that the zeroing of the dosing device is automatically carried out at the same time as each injection is administered.

A further importand advantage of the present device lies in the safety bar against the setting and administering of an excessive dose. This is illustrated in Figure 2. This figure shows how the rear sleeve 10 has been screwed into the front sleeve 5 to a length corresponding to a maximum permitted dose 18, which can be read through the window 15. In this position of the piston rod 16, its lugs 19 rest against the edge of the forward-facing tubular extension 20, which extends forward from the front face of the transversal end wall 12, and no further relative rearward axial movement of the piston rod 16 is possible. If the user tries to continue to move the rear sleeve 10 further into the front sleeve 5, this will have as a consequence that the tubular extension by means of the lugs 19 will push the piston rod 16 forward, and with it also the piston 3 in the cartridge 1. If a needle is connected to the cartridge, the injectable preparation 4 will be expelled from the cartridge 1 without any controlled injection taking place. If no needle is attached, something will ultimately break and the device will be destroyed, but it will not be possible to set an excessive dose.

The lugs 19 may be made resilient to facilitate the insertion of the piston rod 16 through the hole 13 in the transversal end wall 12.

A further important feature is that the thickened cylindrical rear portion 17 of the piston rod 16 should be of such a length that it is essentially flush with and does not protrude from the tubular extension 14 of the rear sleeve 10 when the dosing device is in its zero position. By this, it is prevented that the piston rod is accidentally or intentionally pulled backwards and in its turn pulls the piston 3 in the cartridge 1 backwards. As has been explained in the foregoing, this will disturb the zero setting of the dosing device, and may also lead to air being sucked into the cartridge. This is a further advantageous safety feature of the device of the invention.

Figure 3 shows the device of the invention after a number of doses have been administered. The rear sleeve 10 has now been screwed into the front sleeve 5 for a considerable distance, and the piston rod 16 and the piston 3 have been moved forward in the cartridge. The complete device has thus been considerably shortened, but it will be seen that the setting and administering of a dose is still carried out in the same manner as previously described. This shortening of the device may be regarded as an advantage, as it makes the device handier and easier to carry. It also shows clearly how much of the injectable preparation has been used up. At the same time, the pawl-and-ratchet mechanism has been operative, so that it will not be possible to unscrew the front and rear sleeves 5 and 10 without destroying the device, and said device cannot be reused with a fresh cartridge. This is important from a safety point of view, and is a further advantage of the device of the invention.

It is to be noted that the embodiment of the device shown in the drawings and described in the foregoing is only an example and does not limit the invention. Modifications and alterations are possible without going outside the scope of the appended claims, and this is obvious to a person skilled in the art, once the basic inventive idea has been understood.

Thus, for example, it is possible to arrange the axial grooves 25 in the rear sleeve 10 instead of the front sleeve 5. Correspondingly, the projection or projections 26 will then be arranged inside the front sleeve 5 near its rear end, so that a pawl-and-ratchet mechanism is obtained, which will function in essentially the same way as described in the foregoing. The further modifications necessary for this embodiment are obvious to a person skilled in the art.

The device of the invention is fabricated from materials which are conventionally used in this field, especially then suitable plastic materials, but also such materials as aluminum and other metals, and glass. The selection of a suitable material presents no problem to those skilled in the art. Also the mechanical design in detail lies within the competence of those skilled in the art, taking into account that the device is designed to accept injection cartridges and injection needles or cannulae of a conventional design.

In the foregoing, the device of the invention has been described solely with reference to injection cartridges of the single-chamber type. However, the device may also be used with injection cartridges of the dual-chamber type. In such a case, when the device is to be used for the first time, the user will have to carry out a predetermined number of "dry runs", pushing the rear piston of the cartridge forward to mix the two components of the cartridge, before the first dose is set and administered. After this, the device is operated in the same way as described in the foregoing, and the same advantages will be obtained.

Through the present invention, it has been possible to provide an injection device, which has a number of important advantages which are not known or obvious from the prior art. Thus, through the arrangement of a window through which the dose set can be read, there is only one scale to read, which means that there is less risk that the user will get confused and set an incorrect dose. Also, through the lugs on the piston rod, it will not be possible to set and administer an excessive dose. The tubular extension of the rear sleeve and the matching rear end part of the piston rod also ensure that the piston rod cannot be pulled backwards, and this feature is further enhanced by the pawl-and-ratchet mechanism, which also makes it impossible to unscrew the rear sleeve from the front sleeve and re-use the device with a new injection cartridge. Finally, as the device only consists of three parts of a rather simple design, its costs will be low. This is important, as the device is intended to be disposable, and to be discarded after the cartridge has been emptied.

## Claims

1. A device for dosing and administering one or more injections of an injectable preparation from an injection cartridge, said device comprising:
a tubular front sleeve (5) which encloses an injection cartridge (1) and at its front end is arranged to receive an injection needle to establish a connection with the interior of the cartridge, and at its rear end is provided with an interior thread (9);
a tubular rear sleeve (10), which at its front end is provided with an exterior thread (11) which cooperates with the interior thread (9) of the front sleeve (5), and at its rear end is closed by a transversal wall (12) having a through-going hole (13);
and
a piston rod (16), the front end of which is secured to a piston (3) in the injection cartridge (1), and the rear end of which protrudes through the transversal rear end wall (12) of the rear sleeve, said piston rod (16) being arranged to be moved forward to urge the piston (3) in the injection cartridge (1) forward, thereby to expel said injectable preparation (4) from said cartridge (1) through said needle; and wherein in one of the sleeves (5) are arranged a number of axial grooves (25) with a regular spacing around the periphery of said sleeve, and that in the other sleeve (10) is arranged at least one radial projection (26), which cooperates resiliently with said axial grooves (25) in the first sleeve (5) to act as a pawl-and-ratchet mechanism which allows a relative rotation between the two sleeves in one direction only when the front and rear sleeves are screwed together and which indicates audibly when the two sleeves have been rotated by a given angle in relation to each other, and **characterized** in that said grooves and said at least one projection are arranged in the threaded parts of said sleeves, and that the piston rod (16) is provided with indicating means (18) near its rear end and the rear sleeve (10) is provided with a tubular extension (14) which extends behind the transversal rear end wall (12) and is provided with an opening or a window (15), through which said indicating means (18) on the piston rod (16) can be observed, such that any relative axial movement between the rear sleeve (1) and the piston rod (16) is displayed by said indicating means (18) through said window (15), thereby also indicating the dose set to be administered.

2. A device according to claim 1, **characterized** in that the piston rod (16) is provided with stopping means (19), which are arranged on said piston rod (16) inside said rear sleeve (10) and which cooperate with means (20) arranged inside said rear sleeve (10) to prevent the piston rod (16) and the rear sleeve (10) from being displaced relative to each other by more than a predetermined length in the axial direction.

3. A device according to claim 2, **characterized** in that said stopping means consist of lugs (19), which are arranged on the piston rod (16) and which in a limit position abut against the forward edge of a tubular sleeve (20), which extends forwards from the forward face of the transversal wall (12) for a predetermined length and surrounds the piston rod (16).

4. A device according to any of claims 1-3, **characterized** in that a plurality of projections (26) are arranged with a spacing corresponding to the spacing of the axial grooves (25) in the thread of the corresponding sleeve.

5. A device according to any of claims 1-4, **characterized** in that the axial grooves (25) are arranged in the internal thread of the front sleeve (5), and the projection or projections (26) are arranged near the front end of the external thread of the rear sleeve (10).

6. A device according to any of claims 1-5, **characterized** in that the rear end of the piston rod (16) is provided with a cylindrical portion (17) which fits in the tubular extension (14) of rear sleeve (10) such that is essentially flush with this extension (14) when a zero dose is indicated through the window (15).

## Patentansprüche

1. Vorrichtung zum Dosieren und Handhaben einer oder mehrerer Injektionen einer injizierbaren Zubereitung von einer Injektionspatrone, wobei die Vorrichtung aufweist:
eine rohrförmige Vorderhülse (5), die eine Injektionspatrone (1) umschließt und wobei ihr Vorderende zur Aufnahme einer Injektionsnadel ausgebildet ist, um eine Verbindung mit dem Inneren der Patrone herzustellen, und ihr Rückende mit einem Innengewinde (9) versehen ist,
eine rohrförmige Rückhülse (10), deren Vorderende mit einem Außengewinde (11) versehen ist, das mit dem Innengewinde (9) der Vorderhülse (5) zusammenwirkt und deren Rückende durch eine Querwand (12) verschlossen ist, die ein Durchgangsloch (13) aufweist,
und einem Kolbenstab (16), dessen Vorderende mit einem Kolben (3) in der Injektionspatrone (11) verbunden ist und dessen Rückende durch die Rückend-Querwand (12) der Rückhülse vorsteht, wobei der Kolbenstab (16) vorwärts zum Vorwärtszwängen des Kolbens (3) in der Injektionspatrone (1) bewegbar ist und dabei die injizierbare Zubereitung (4) aus der Patrone (1) durch die Nadel ausstößt, und wobei in einer der Hülsen (5) eine Anzahl von Axialnuten (25) mit regelmäßigen Abständen entlang des Umfangs der Hülse angeordnet sind, und daß in der anderen Hülse (10) zumindest ein Radialvorsprung (26) angeordnet ist, der nachgiebig mit den Axialnuten (25) in der ersten Hülse (5) zusammenwirkt, um als Ratschenmechanismus zu arbeiten, der eine Relativdrehung zwischen den beiden Hülsen in einer Richtung nur dann erlaubt, wenn die Vorder- und die Rückhülse miteinander verschraubt sind, und der hörbar anzeigt, wenn die beiden Hülsen um einen vorgegebenen Winkel in Bezug aufeinander gedreht wurden,
dadurch **gekennzeichnet,** daß die Nuten und der zumindest eine Vorsprung in den Gewindeteilen der Hülsen angeordnet sind und daß der Kolbenstab (16) mit Anzeigemitteln (18) nahe seinem Rückende versehen ist und die Rückhülse (10) mit einer rohrförmigen Verlängerung (14) versehen ist, die sich hinter die Rückendquerwand (12) erstreckt und mit einer Öffnung oder einem Fenster (15) versehen ist, durch das die Anzeigemittel (18) auf dem Kolbenstab (16) beobachtet werden können, so daß jede relative Axialbewegung zwischen der Rückhülse (1) und dem Kolbenstab (16) von den Anzeigemitteln (18) durch das Fenster (15) angezeigt wird, wodurch somit die zu handhabende eingestellte Dosis angezeigt wird.

2. Vorrichtung nach Anspruch 1,
dadurch **gekennzeichnet,** daß der Kolbenstab (16) mit Anschlagmitteln (19) versehen ist, die auf dem Kolbenstab (16) innerhalb der Rückhülse (10) angeordnet sind und mit Mitteln (20) innerhalb der Rückhülse (10) zusammenwirken, um zu verhindern, daß der Kolbenstab (16) und die Rückhülse (10) relativ zueinander um mehr als eine vorgegebene Länge in Axialrichtung verschoben werden.

3. Vorrichtung nach Anspruch 2,
dadurch **gekennzeichnet,** daß die Anschlagsmittel aus Vorsprüngen (19) bestehen, die auf dem Kolbenstab (16) angeordnet sind und die in einer Beschränkungsposition gegen die Vorderkante einer rohrförmigen Hülse (20) anschlagen, die sich von der Vorderfläche der Querwand (12) um eine vorgegebene Länge nach vorn erstreckt und den Kolbenstab (16) umgibt.

4. Vorrichtung nach einem der Ansprüche 1 bis 3,
dadurch **gekennzeichnet,** daß eine Anzahl von Vorsprüngen (26) mit einem Abstand angeordnet ist, der dem Abstand der Axialnuten (25) im Gewinde der entsprechenden Hülse entspricht.

5. Vorrichtung nach einem der Ansprüche 1 bis 4,
dadurch **gekennzeichnet,** daß die Axialnuten (25) im Innengewinde der Vorderhülse (5) und der oder die Vorsprünge (26) nahe dem Vorderende des Außengewindes der Rücknut (10) angeordnet sind.

6. Vorrichtung nach einem der Ansprüche 1 bis 5,
dadurch **gekennzeichnet,** daß das Rückende des Kolbenstabes (16) mit einem zylindrischen Abschnitt (17) versehen ist, der in die rohrförmige Verlängerung (14) der Rückhülse (10) derart einpaßt, daß er im wesentlichen mit dieser Verlängerung (14) fluchtet, wenn durch das Fenster (15) im wesentlichen eine Nulldosis angezeigt wird.

## Revendications

1. Dispositif pour le dosage et l'administration d'une ou de plusieurs injections d'une préparation injectable à partir d'une cartouche d'injection, ce dispositif comprenant :
une enveloppe tubulaire avant (5) qui renferme une cartouche d'injection (1) et qui, sur son extrémité avant, est apte à recevoir une aiguille d'injection établissant une communication avec l'intérieur de la cartouche, et qui, sur son extrémité arrière, est dotée d'un filetage interne (9);
une enveloppe tubulaire arrière (10) qui, sur son extrémité avant, présente un filetage extérieur (11) qui coopère avec le filetage intérieur (9) de l'enveloppe avant (5), et qui, sur son extrémité arrière, est obturée par une paroi transversale (12) avec un trou traversant (13) ; et
une tige de piston (16), dont l'extrémité avant est fixée sur un piston (3) dans la cartouche d'injection (1), et dont l'extrémité arrière fait saillie par la paroi d'extrémité arrière transversale (12) de l'enveloppe arrière, la tige de piston (16) pouvant se déplacer vers l'avant pour solliciter le piston (3) dans la cartouche d'injection (1) sur l'avant, permettant ainsi d'expulser la préparation injectable (4) à partir de la cartouche (1) à travers l'aiguille ; et dispositif dans lequel, dans l'une des enveloppes (5), il est ménagé un certain nombre de gorges axiales (25) avec un espacement régulier sur la périphérie de l'enveloppe et en ce que dans l'autre enveloppe (10) est disposée au moins une saillie radiale (26) qui coopère de façon élastique avec les gorges axiales (25) dans la première enveloppe (5) pour servir de mécanisme de verrouillage à cliquet permettant une rotation relative entre les deux enveloppes dans une direction seulement lorsque les enveloppes avant et arrière sont vissées ensemble et qui indique de façon audible le moment où les deux enveloppes ont effectué une rotation selon un angle donné l'une par rapport à l'autre, et caractérisé en ce que les gorges et au moins une saillie sont disposées dans les parties filetées des enveloppes et en ce que la tige de piston (16) est dotée de moyens d'indication (18) à proximité de son extrémité arrière et en ce que l'enveloppe arrière (10) est munie d'un prolongement tubulaire (14) qui s'étend derrière la paroi d'extrémité arrière transversale (12) et comporte une ouverture ou une fenêtre (15) par laquelle on peut observer les moyens indicateurs (18) sur la tige de piston (16) de telle sorte que tout mouvement axial relatif entre l'enveloppe arrière (1) et la tige de piston (16) est affiché par les moyens indicateurs (18) à travers la fenêtre (15), indiquant ainsi également la dose fixée qu'il faut administrer.

2. Dispositif selon la revendication 1, caractérisé en ce que la tige de piston (16) est dotée de moyens de butée (19) qui sont disposés sur la tige de piston (16) à l'intérieur de l'enveloppe arrière (10) et qui coopèrent avec des moyens (20) disposés à l'intérieur de l'enveloppe arrière (10) pour empêcher la tige de piston (16) et l'enveloppe arrière (10) d'être déplacées l'une par rapport à l'autre sur une longueur supérieure à la longueur prédéterminée dans la direction axiale.

3. Dispositif selon la revendication 2, caractérisé en ce que les moyens de butée consistent en des oeillets (19) qui sont disposés sur la tige de piston (16) et qui dans une position limite viennent en butée contre le bord avant de l'enveloppe tubulaire (20) et qui s'étendent vers l'avant à partir de la face avant de la paroi transversale (12) sur une longueur prédéterminée et entourent la tige de piston (16).

4. Dispositif selon l'une quelconque des revendications 1-3, caractérisé en ce que plusieurs saillies (26) sont disposées selon un espacement correspondant à l'espacement des gorges axiales (25) dans le filetage de l'enveloppe correspondante.

5. Dispositif selon l'une quelconque des revendications 1-4, caractérisé en ce que les gorges axiales (25) sont disposées dans le filetage interne de l'enveloppe avant (5) et la saillie ou les saillies (26) sont disposées à proximité de l'extrémité avant du filetage externe de l'enveloppe arrière (10).

6. Dispositif selon l'une quelconque des revendications 1-5, caractérisé en ce que l'extrémité arrière de la tige de piston (16) est dotée d'une portion cylindrique (17) qui s'engage dans le prolongement tubulaire (14) de l'enveloppe arrière (10) de façon à se situer sensiblement en affleurement sur ce prolongement (14) lorsqu'une dose zéro est indiquée par la fenêtre (15).
